# EUROPEAN PATENT SPECIFICATION

(11) **EP 2 364 666 B1**
(45) Date of publication and mention of the grant of the patent: **13.06.2018**
(21) Application number: 11250257.0
(22) Date of filing: 07.03.2011
(51) Int. Cl.: A61B 17/072, A61B 17/29, A61B 90/00

(54) **System for determining and adjusting positioning and orientation of a surgical device**
System zur Bestimmung und Einstellung der Position und Ausrichtung einer chirurgischen Vorrichtung
Système pour déterminer et ajuster le positionnement et l'orientation d'un dispositif chirurgical

(30) Priority: 08.03.2010 US 311411 P; 10.02.2011 US 201113024583
(43) Date of publication of application: 14.09.2011
(73) Proprietor: Covidien LP, Mansfield, MA 02048 (US)
(72) Inventor: Ma, Youg, Cheshire, CT 06410 (US)
(74) Representative: Soames, Candida Jane

(56) References cited:
- EP-A1- 1 915 966
- EP-A1- 2 044 890
- EP-A2- 1 915 957
- WO-A1-02/34147
- WO-A2-2008/042423
- US-A1- 2008 188 868
- US-B1- 6 799 065

## Description

The present disclosure relates to a surgical device and, more particularly, to a system and method for sensing angular motion of the surgical device in order to adjust positioning and/or orientation of the surgical device.

In laparoscopic procedures, surgery may be performed in the interior of the abdomen through a small incision. In endoscopic procedures, surgery may be performed in any hollow viscus of the body through narrow endoscopic tubes inserted through small entrance wounds in the skin. Laparoscopic and endoscopic procedures generally require that any instrumentation inserted into the body be sealed, i.e., provisions must be made to ensure that gases do not enter or exit the body through the laparoscopic or endoscopic incision. Moreover, laparoscopic and endoscopic procedures often require the surgeon to act on organs, tissues, and vessels far removed from the incision, thereby requiring that any instruments be used in such procedures be long and narrow while being functionally controllable from one end of the instrument.

In medical science, a precise determination of the position of an applied medical instrument in various diagnostic and therapeutic methods is necessary. Instruments of this kind, for example, may be intravascular catheters, guidance wires, biopsy needles, minimally invasive surgical instruments or the like. However, conventional techniques and tools for determining the spatial positioning and/or orientation of a medical instrument are inaccurate and error-prone.

The reader may be further enlightened as to the state of the art by reference to the publications US 6 799 065, EP 2 044 890, EP 1 915 966 and EP 1 915 957. The present invention seeks to alleviate at least some of the technical problems associated with the prior art of US 6 799 065 and EP 2 044 890 and accordingly provides a surgical instrument according to claim 1 herein.

In accordance with the present disclosure, a surgical instrument is provided. The surgical instrument includes an elongate member having a proximal end and a distal end, the elongate member defining a longitudinal axis and configured to rotate relative to the longitudinal axis. The surgical instrument further includes a handle member configured to be attached to the proximal end of the elongate member, the handle member operatively associated with a rotation mechanism. The surgical instrument also includes an end effector configured to be attached to the distal end of the elongate member, the end effector operatively associated with an articulation mechanism and configured to be pivotable throughout a plurality of directions relative to the longitudinal axis of the elongate member. A software algorithm may be used to identify, track, and control directional movement of the end effector in accordance with rotational movement of the elongate member.

In one embodiment, the end effector includes a pair of jaw members.

In yet another embodiment, the plurality of directions include vertical displacements and horizontal displacements, the displacements driven by one or more actuators positioned within the handle member. In another embodiment, the handle member includes a drive assembly for actuating the directional movement of the end effector and the rotational movement of the elongate member.

The software algorithm records a first position of the elongate member, a first position of the end effector, and establishes a relationship between the first position of the elongate member and the first position of the end effector. When the elongate member rotates to a second position, the software algorithm records the second position of the elongate member and compares the second position of the elongate member to the first position of the end effector. Additionally, the software algorithm records all position changes of the elongate remember relative to the end effector and, upon an operator command, repositions the end effector and the elongate member to match a predetermined positional reference point.

In another embodiment, the software algorithm uses triangulation techniques to reposition the end effector to match a predetermined positional reference point of the elongate member. The triangulation techniques involve using one or more angle encoders. The one or more angle encoders are magnetic sensors or potentiometers.

In yet another embodiment, the software algorithm is controlled via a control panel positioned about the handle member. Additionally, the software algorithm may be wirelessly controlled via an external source.

An exemplary method of using a surgical instrument is also provided in accordance with the present disclosure. The method includes providing an elongate member having a proximal end and a distal end, the elongate member defining a longitudinal axis and configured to rotate relative to the longitudinal axis, attaching a handle member to the proximal end of the elongate member, the handle member operatively associated with a rotation mechanism, and attaching an end effector to the distal end of the elongate member, the end effector operatively associated with an articulation mechanism and configured to be pivotable throughout a plurality of directions relative to the longitudinal axis of the elongate member. A software algorithm may be used to identify, track, and control directional movement of the end effector in accordance with rotational movement of the elongate member.

### BRIEF DESCRIPTION OF THE DRAWINGS

Various embodiments of the presently disclosed surgical instrument are described hereinbelow with references to the drawings, wherein:
FIG. 1 is a perspective view of a surgical instrument for applying surgical staples to attach objects to surgical tissue, in accordance with the present disclosure;
FIG. 2 is a perspective view of a distal end of the surgical instrument of FIG. 1, in accordance with the present disclosure;
FIG. 3 is a cross-sectional view taken along lines 3-3 of FIG. 1, illustrating the handle mechanism of the surgical instrument of FIG. 1, in accordance with the present disclosure;
FIG. 4 is a diagram illustrating the directional movement of the end effector in accordance with rotational movement of the elongate member, in accordance with the present disclosure; and
FIG. 5 is a flowchart illustrating an example process of positioning and/or orienting the surgical instrument of FIG. 1, in accordance with the present disclosure.

### DETAILED DESCRIPTION OF THE EMBODIMENTS

In the example embodiments of the present disclosure, a tracking system may provide positioning and/or orientation information of the medical instrument with respect to the patient or a reference coordinate system. A medical practitioner may refer to the tracking system to ascertain the position of the medical instrument when the instrument may not be within the practitioner's line of sight or when the instrument has been moved from the initial reference point. The medical practitioner may use the tracking system to determine when the instrument may be positioned in a preferred location.

A more particular description of the present disclosure, briefly summarized above, may be had by reference to the embodiments of the present disclosure described in the present specification and illustrated in the appended drawings. It is to be noted, however, that the specification and appended drawings illustrate only certain embodiments of this present disclosure and are, therefore, not to be considered limiting of its scope. The present disclosure may admit to equally effective embodiments.

Reference will now be made in detail to exemplary embodiments of the present disclosure. While the present disclosure will be described in conjunction with these embodiments, it is to be understood that the described embodiments are not intended to limit the present disclosure solely and specifically to only those embodiments. On the contrary, the present disclosure is intended to cover alternatives, modifications, and equivalents that may be included within the scope of the present disclosure as defined by the attached claims.

In the following description, numerous specific details are set forth in order to provide a thorough understanding of the present disclosure. It will be apparent to one skilled in the art, however, that the present disclosure may be practiced without these specific details.

In other instances, well-known circuits, control logic, and the details of computer program instructions for conventional algorithms and processes have not been shown in detail in order not to obscure the present disclosure unnecessarily. For instance, software programming code, which embodies aspects of the present disclosure, may be maintained in permanent storage, such as a computer readable medium. In a client/server environment, such software programming code may be stored on a client or a server. The software programming code may be embodied on any of a variety of known media for use with a data processing system, such as a diskette, or hard drive, or CD-ROM. The code may be distributed on such media, or may be distributed to users from the memory or storage of one computer system over a network of some type to other computer systems for use by users of such other systems.

Additionally, arrangement of components to achieve the same functionality may be effectively "operably coupled" or "coupled" or "in communication with" or "communicates with" or "operatively communicate" such other objects that the desired functionality may be achieved. Hence, any two components herein combined to achieve a particular functionality may be seen as associated with each other such that the desired functionality may be achieved, irrespective of architectures or intermodal components. Likewise, any two components so associated may also be viewed as being "connected," or "attached," to each other to achieve the desired functionality, and any two components capable of being so associated may also be viewed as being "operably couplable," to each other to achieve the desired functionality.

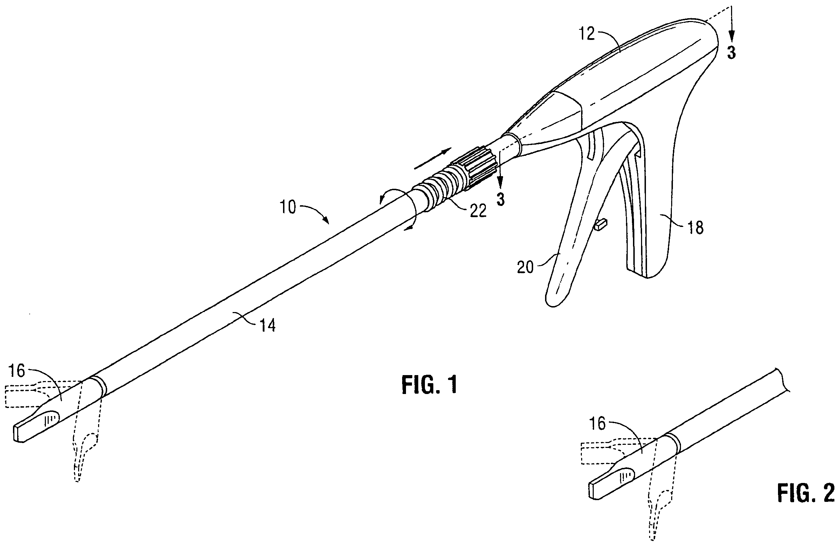

With reference to FIG. 1, a perspective view of a surgical instrument for applying surgical staples to attach objects to surgical tissue, in accordance with the present disclosure is presented.

The apparatus/instrument 10 may be particularly adapted for endoscopic application of surgical staples to attach surgical mesh to body tissue during hernia repair. However, one skilled in the art may contemplate using apparatus 10 in a plurality of surgical and non-surgical applications. Except where noted otherwise, the materials utilized in the components of the apparatus 10 generally include such materials as polycarbonate for housing sections and related components, and stainless steel for such components which transmit forces.

The apparatus 10 may include handle portion 12 and endoscopic section 14 having at the distal end portion a staple storage magazine 16 which pivots with respect to at least one side of the longitudinal axis extending centrally through the endoscopic section. Generally, staple storage magazine 16 may selectively pivot up to about 45 degrees with respect to the aforesaid longitudinal axis. The staple storage magazine 16 is shown in general alignment with the longitudinal axis of the endoscopic section and in phantom to illustrate a range of movement. The total range of pivotal motion of the staple storage magazine 16 as shown is approximately 90 degrees, i.e. 45 degrees to each side of neutral. The endoscopic section 14 may be referred to as an elongate member having a proximal end and a distal end, the elongate member defining a longitudinal axis and configured to rotate relative to the longitudinal axis.

The handle 12 of instrument 10 may include manual grip 18 and pivotal trigger 20 which may be pivoted toward and away from manual grip 18. Trigger 20 may be pivoted toward manual grip 18 during the staple advancing and firing sequence. Trigger 20 may pivot away from manual grip 18 to return the apparatus or surgical instrument 10 to the pre-fired condition in position for firing the staple next in line. The handle member 12 may be configured to be attached to the proximal end of the elongate member, the handle member 12 operatively associated with a rotation mechanism described below.

A double knurled finger operative collar 22 may be rotatable and adapted to rotate the entire endoscopic section 14 a full 360 degrees, while proximal movement of the collar 22 may produce pivotal motion of the staple storage magazine to one of the positions shown in phantom in FIG. 1. To achieve the other position shown in phantom in FIG. 1, the collar 22 may be rotated 180 degrees thereby rotating the entire endoscopic section and causing the position of the magazine 16 to be reversed to the other position shown in phantom. Thus, the combination of full rotation of the endoscopic section 14 and the pivotal movement of the staple storing magazine may facilitate a wide range of articulation of the distal end of the staple magazine 16, thus facilitating application of staples over a wide range of locations (±180 degrees) and in any of a plurality of orientations/directions.

When the collar 22 is moved to its proximal-most position the staple magazine may be in one of the positions shown in phantom in FIG. 1, i.e., at an angle with respect to the longitudinal axis of the instrument 10. When the collar 22 is advanced to the distal-most position the staple magazine may assume the position shown in FIG. 1, i.e., in alignment with the longitudinal axis of the instrument 10.

The full 90 degrees of movement of the magazine 16 may be achieved by longitudinal movement of collar 22 in combination with full rotation of the endoscopic section 14. The longitudinal movement of collar 22 causes pivotal movement of the staple storing magazine to 45 degrees in one direction and rotation of the endoscopic section 14 provides completion of the articulation of the magazine. Both of these movements in combination, facilitate a wide range of maneuverability of the distal end of the staple magazine 16, thus facilitating application of staples over a wide range of locations (±180 degrees) and in any of a plurality of orientations/directions, as described in more detail below with reference to FIG. 2. Thus, the end effector may be configured to be attached to the distal end of the elongate member, the end effector operatively associated with an articulation mechanism and configured to be pivotable throughout a plurality of directions relative to the longitudinal axis of the elongate member. The end effector may include a pair of jaw members.

Additionally, the plurality of directions may include vertical displacements and horizontal displacements, the displacements driven by one or more actuators positioned within the handle member. Also, the handle member 12 may include a drive assembly (not shown) for actuating the directional movement of the end effector and the rotational movement of the elongate member.

With reference to FIG. 2, a perspective view of a distal end of the surgical instrument of FIG. 1, in accordance with the present disclosure is presented.

Alternatively, the positions of the staple storing magazine 16 may be achieved as shown in FIG. 2, i.e., by movement of the magazine 16 between zero degrees and about 45 degrees on either side of the longitudinal axis. In such arrangement, to achieve the positions shown in phantom in FIG. 2, the collar 22 (see FIG. 1) may be moved distally and proximally, equal distances on either side of a neutral detent. Movement in one direction would pivot the magazine 16 to one side and movement in the other direction would cause pivotal movement of the magazine 16 in the opposite direction. The directions selected would be arbitrary. However, in this last described embodiment the orientation of the magazine 16 would be the same throughout the 90 degree pivoting range, whereas in the embodiment of FIG. 1, the orientation of the magazine when on one side may be opposite the orientation when on the other.

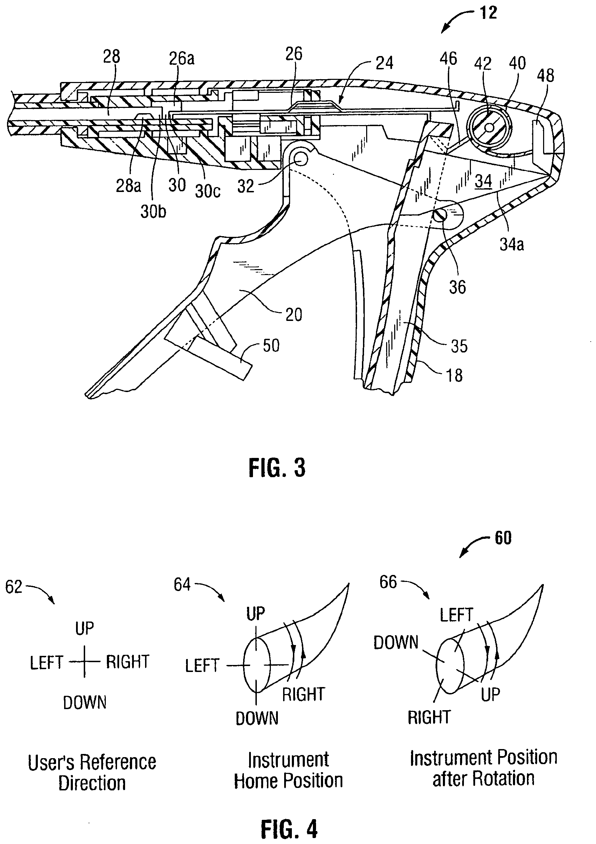

With reference to FIG. 3, a cross-sectional view taken along lines 3-3 of FIG. 1, illustrating the handle mechanism of the surgical instrument of FIG. 1, in accordance with the present disclosure is presented.

The handle mechanism 12 provides controlled distal movement to the pusher assembly 24, a portion of which is shown in FIG. 3. The pusher assembly 24 may extend through the endoscopic section 14 (see FIG. 1). The surgical instrument 10 may be entirely disposable. However, it is also contemplated and within the scope of the present disclosure to construct the endoscopic section 14 to be selectively detachable whereby the handle 12 may be sterilized and reused, or the endoscopic section 14 may be sterilized, and the staple storage magazine 16 re-loaded with staples for re-use. Alternatively a replacement staple magazine, and optionally a replacement endoscopic section, may be detachably secured to a disposable handle 12 for multiple uses during a single surgical procedure. Thus, any combination of alternatives may be incorporated within the scope of the present disclosure.

In operation, pusher assembly 24 may include flanged thrust bar 26 connected to firing rod 28 by lost motion connector 30 as shown in FIG. 3. Lost motion connector 30 may be a bar having a generally "U-shaped" configuration. The lost motion connector 30 may provide a positive connection between flanged thrust bar 26 and firing rod 28, yet may also provide a small space between the firing rod and the thrust bar 26. Since the respective slots 28*a* and 26*a* in the firing rod 28 and in the thrust bar 26 may be dimensioned slightly larger in width than the thickness of the legs 30*b* and 30*c* of the lost motion connector 30 which may be received in these slots, a small degree of relative movement may be permitted between the components in the staple firing chain.

Trigger mechanism 20 ma y be pivotally attached at pivot pin 32 for pivotal movement toward and away from handle grip 18, and may be adapted to produce upward and downward rotational movement of triangular member 34 when horizontal pin 36, attached to trigger mechanism 20, traverses an upward arc whose center of rotation may be located at pivot pin 32. Thus, when handle grip 18 is positioned in the palm of the user's hand and trigger mechanism 20 is squeezed toward handle grip 18, horizontal pin 36 may traverse an upward arc while engaging the longer side 34*a* of triangular member 34. This movement may cause triangular member 34 to rotate upward in a counterclockwise direction while upright member 35 to which it may be attached, may pivot forwardly about a point of rotation defined by pivot pin 37 located at the lowermost end of a handle grip 18.

Additionally, in operation, pusher assembly 24 may be connected to upright member 35 such that inward squeezing of trigger mechanism 20 may cause the entire pusher assembly to advance distally against the constant force provided by negator spring 40. The negator spring 40 may be formed of a resilient flat spring material coiled about the rotational bar 42, which may be rotationally mounted about cross member (not shown) which forms part of bracket 46. The free end of negator spring 40 may be attached to an anchor pin 48, while the spring 40 may be biased toward the coiled configuration.

It may therefore be appreciated that after squeezing trigger mechanism 20, release of the trigger mechanism may permit the negator spring 40 to assume control and to return rotational bar 42 to the pre-fired proximal location by the automatic winding action of the negator spring 40 to its original unloaded configuration. This motion in turn may cause the entire pusher assembly 24 to return to the proximal-most pre-fired position. The constant force of negator spring 40 may prevent the natural tendency of the user to rotate the hand as with springs which increase in force when progressing through a full spring cycle.

Trigger stop device 50 may be attached to trigger mechanism 20 and may be configured and dimensioned for engagement with handle grip 18 in a manner to thereby limit the proximal pivotal movement of trigger mechanism 20. Depending upon the particular limits required in the apparatus, trigger stop device 50 may be dimensioned accordingly. As a result, FIG. 3 illustrates how the surgical instrument 10 includes two modes of operation. That is, a rotational mode of operation and an articulation mode.

With reference to FIG. 4, a diagram illustrating the directional movement of the end effector in accordance with rotational movement of the elongate member, in accordance with the present disclosure is presented.

In FIG. 4, the diagram 60 shows a user's reference location 62, a surgical instrument reference position 64 (i.e., home position), and a surgical instrument position after rotation 66. As used herein, the term "location" may refer to the spatial coordinates of an object, the term "orientation" may refer to angular coordinates of the object, and the term "position" may refer to the full positional information of the object, comprising both location and orientation coordinates.

The surgical instrument 10 (see FIG. 1) with the rotating shaft 14 may be controlled by electronics or control circuitry to move the distal end or end effector of the surgical instrument 10 in a plurality of radial directions. These directions may be discrete directions, such as up, down, left, right. Each discrete direction may be achieved by a cable or actuator (not shown). While the shaft 14 of the surgical instrument 10 rotates, the cable or actuator rotates with it. The operator of the surgical instrument 10 may send commands to articulate the end effector. After the shaft 14 rotates away from its initial position, the control on the operator's side may become counter-intuitive. For example, if the shaft rotates 90 degrees off the initial position, up-down control may end up being left-right movement of the end effector. That is, the physical driving direction of an instrument 10 does not match the operator's intuitive direction due to the rotation of the surgical instrument 10. This may cause the surgeon to mishandle the surgical instrument and may cause inadvertent operation of the surgical instrument.

Thus, in order to resolve such issues and in accordance with diagram 60, once the locations of the reference points are determined, a processor may execute software which repositions the surgical instrument 10 to reflect the initial position of the surgical instrument 10. The directional movement may be controlled by a software algorithm, which in turn may be executed via control circuitry. In certain embodiments, control circuitry may be employed to control or regulate various isolated or inter-connected components. As used herein the term "control circuitry" may include, but is not limited to, electrical circuitry, regulators, valves, rheostats, silicon chips, resistors, capacitors, transistors etc., that may maintain or regulate over all control or partial control over some component parts or systems. In one embodiment, control circuitry may process input and output signals from individual or interlinked components. Moreover, the control circuitry in association with the software algorithm may be used to identify, track, and control directional movement of the end effector in accordance with rotational movement of the elongate member.

In an alternative embodiment, sensors may be used to determine the position and/or orientation of the surgical instrument 10 relative to an anatomical part(s) and/or relative to the position of a user(s) of the surgical instrument 10. The sensors may provide information regarding, inter alia, the physical boundary limitations of an operating field or movement of the surgical instrument 10 with respect to the patient. The information may include, but is not limited to, boundary sensing and sensory signals. Moreover, a user includes, but is not limited to a surgeon, operating room personnel, surgical trainer or a robotic user. In some alternative embodiments, sensory signals may be delivered to a surgical instrument 10 or a human or robotic user either through a direct hardwired system or through a wireless system. Those skilled in the art will recognize that signals may be conveyed through numerous means. For instance, the means for signal communication may include, but is not limited to radio frequencies, acoustic, ultrasound, electromagnetic, infrared, optical, etc.

Back to FIG. 4, the user of the surgical instrument 10 (see FIG. 1) may reorient or reposition the surgical instrument 10 to a desired location via the click of a button. In other words, the surgical instrument 10 may receive and act upon one or more control commands. The control commands may be enabled via one or more buttons or remotely through a wired or wireless media. Thus, the software algorithm may be controlled via a control panel positioned about the handle member 12 or may be wirelessly controlled via one or more external sources, such as a computing device (e.g., computer, mobile device or any electronic device connected to a network). The external device may be positioned in a remote location relative to the surgical instrument 10.

User initiated commands may include, but are not limited to, instructions either to activate or inactivate a surgical instrument 10. User-initiated commands may also include instructions to reorient or reposition the surgical instrument 10. Those skilled in the art will recognize that the activation or inactivation may occur within or outside a physical boundary limitation of an operating field.

Furthermore it is conceivable by those skilled in the art that user-initiated commands may include auto-inactivation or auto-activation of the surgical instrument 10 may occur abruptly or at regularly phased intervals. In some embodiments, the activation or the inactivation of the surgical instrument 10 may occur through modification of one or more operative characteristics of the surgical instrument 10. Additionally or alternatively, the activation or the inactivation may occur while the surgical instrument 10 may be at least partly functioning within an operating field. Also, in some instances, user initiated commands may instruct the surgical instrument 10 to slow down or speed up or change directions. Those skilled in the art will appreciate that numerous methods, protocols, procedures or algorithms are available.

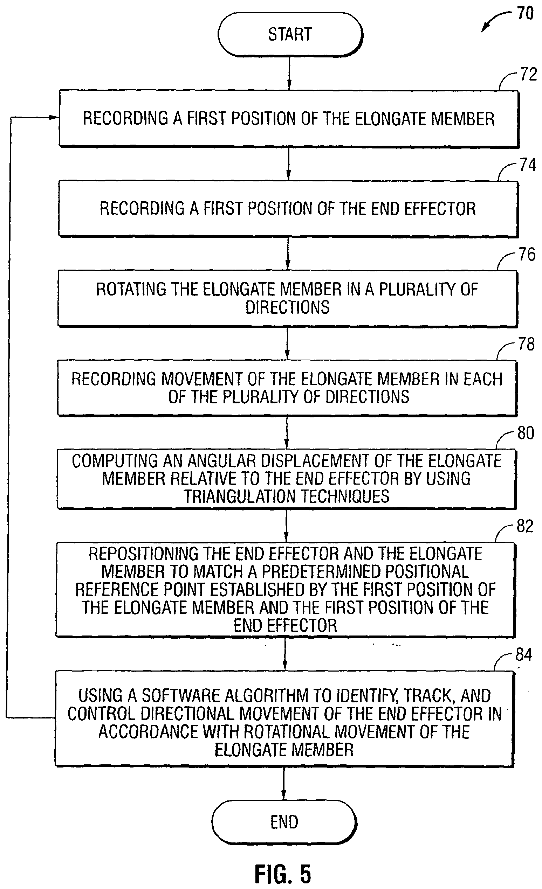

Thus, in accordance with the present disclosure, a software algorithm may be used to allow the end effector to match the commands of the operator. The software algorithm may include the following process described with reference to FIG. 5.

With reference to FIG. 5, a flowchart 70 illustrating an example process of orienting the surgical instrument of FIG. 1, in accordance with the present disclosure is presented.

In step 72, a first position of the elongate member may be recorded. In step 74, a first position of the end effector may be recorded. In step 76, the elongate member may be rotated in a plurality of positions. In step 78, the movement of the elongate member may be recorded in each of the plurality of directions. In step 80, an angular displacement of the elongate member relative to the end effector may be computed by using triangulation techniques. In step 82, repositioning of the end effector and the elongate member to match a predetermined positional reference point established by the first position of the elongate member and the first position of the end effector takes place. In step 84, a software algorithm may be used to identify, track, and control directional movement of the end effector in accordance with rotational movement of the elongate member. The process then ends for the first cycle or first iteration. However, the process may be a continuous iterative process. In other words, the steps of the process may repeat for a number cycles or iterations, where the recording, rotating, computing, readjusting, and repositioning steps are constantly repeated.

The illustrated devices or methods may be implemented in software, hardware, firmware or combinations thereof. The steps discussed herein need not be performed in the stated order. Several of the steps could be performed concurrently with each other. Furthermore, if desired, one or more of the above described steps may be optional or may be combined without departing from the scope of the present disclosure.

In other words, the software algorithm records a first position of the elongate member, a first position of the end effector, and establish a relationship between the first position of the elongate member and the first position of the end effector. Then, when the elongate member rotates to a second position, the software algorithm may record the second position of the elongate member and compare the second position of the elongate member to the first position of the end effector. This angular shift may be stored in one or more storage units described below. Furthermore, the software algorithm records all position changes of the elongate remember relative to the end effector and, upon an operator command, reposition the end effector and the elongate member to match a predetermined positional reference point. The software algorithm may use triangulation techniques to reposition the end effector to match a predetermined positional reference point of the elongate member. The triangulation techniques may involve one or more angle encoders, such as, magnetic sensors or potentiometers.

In an alternative embodiment, storage units may be used to record the directional movement of the end effector in accordance with rotational movement of the elongate member (i.e., endoscopic section 14). The storage units may include any desired type of volatile and/or non-volatile memory such as, for example, static random access memory (SRAM), dynamic random access memory (DRAM), flash memory, read-only memory (ROM), etc. The storage units may include any desired type of mass storage device including hard disk drives, optical drives, tape storage devices, etc.

Those skilled in the art will recognize that the various aspects described herein which may be implemented, individually or collectively, by a wide range of hardware, software, firmware, or any combination thereof may be viewed as being composed of various types of "electrical circuitry." Consequently, as used herein "electrical circuitry" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes or devices described herein, or a microprocessor configured by a computer program which at least partially carries out processes or devices described herein), electrical circuitry forming a memory device (e.g., forms of random access memory) or electrical circuitry forming a communications device (e.g., a modem, communications switch, or optical-electrical equipment). Those having skill in the art will recognize that the subject matter described herein may be implemented in an analog or digital fashion or some combination thereof.

While various aspects and embodiments have been disclosed herein, other aspects and embodiments will be apparent to those skilled in the art. The various aspects and embodiments disclosed herein are for purposes of illustration and are not intended to be limiting, with the true scope being indicated by the following claims.

The foregoing detailed description has set forth various embodiments of the devices or processes via the use of flowcharts, diagrams, figures or examples. Insofar as such flowcharts, diagrams, figures or examples contain one or more functions or operations, it will be understood by those within the art that each function or operation within such flowchart, diagram, figure or example may be implemented, individually or collectively, by a wide range of any combination thereof.

From the foregoing and with reference to the various figure drawings, those skilled in the art will appreciate that certain modifications may also be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. A surgical instrument (10), comprising:
an elongate member (14) having a proximal end and a distal end, the elongate member (14) defining a longitudinal axis and configured to rotate relative to the longitudinal axis;
a handle member (12) configured to be attached to the proximal end of the elongate member (14), the handle member (12) operatively associated with a rotation mechanism; and
an end effector configured to be attached to the distal end of the elongate member (14), the end effector operatively associated with an articulation mechanism and configured to be pivotable throughout a plurality of directions relative to the longitudinal axis of the elongate member (14);
wherein a software algorithm running on a processor records a first position of the elongate member, a first position of the end effector, and establishes a predetermined positional reference point which is a relationship between the first position of the elongate member and the first position of the end effector, wherein the software algorithm further records all position changes of the elongate member relative to the end effector and, upon operator command, repositions the end effector and the elongate member to match the predetermined positional reference point.

2. The surgical instrument (10) according to Claim 1, wherein the end effector includes a pair of jaw members.

3. The surgical instrument (10) according to Claim 1 or Claim 2, wherein the plurality of directions include vertical displacements and horizontal displacements, the displacements driven by one or more actuators positioned within the handle member (12).

4. The surgical instrument (10) according to any preceding claim, wherein the handle member (12) includes a drive assembly for actuating the directional movement of the end effector and the rotational movement of the elongate member (14).

5. The surgical instrument (10) according to Claim 1, wherein when the elongate member (14) rotates to a second position, the software algorithm records the second position of the elongate member (14) and compares the second position of the elongate member (14) to the first position of the end effector.

6. The surgical instrument (10) according to any preceding claim, wherein the software algorithm uses triangulation techniques to reposition the end effector to match a predetermined positional reference point of the elongate member (14), preferably wherein the triangulation techniques involve using one or more angle encoders, preferably still wherein the one or more angle encoders are magnetic sensors or potentiometers.

7. The surgical instrument (10) according to any preceding claim, wherein the software algorithm is controlled via a control panel positioned about the handle member (12).

8. The surgical instrument (10) according to any preceding claim, wherein the software algorithm is wirelessly controlled via an external source.

9. The surgical instrument (10) according to Claim 8, wherein the external source is a computing device, preferably wherein the external source is a computing device positioned in a remote location.

## Patentansprüche

1. Chirurgisches Instrument (10), umfassend:
ein längliches Element (14), das ein proximales Ende und ein distales Ende aufweist, wobei das längliche Element (14) eine longitudinale Achse definiert und eingerichtet ist, um relativ zu der longitudinalen Achse zu rotieren;
ein Griffelement (12), eingerichtet, um an dem proximalen Ende des länglichen Elements (14) angebracht zu werden, wobei das Griffelement (12) operativ mit einem Rotationsmechanismus assoziiert ist; und
einen Endeffektor, eingerichtet, um an dem distalen Ende des länglichen Elements (14) angebracht zu werden, wobei der Endeffektor operativ mit einem Gelenkverbindungsmechanismus assoziiert ist und eingerichtet, um schwenkbar durch eine Vielzahl von Richtungen relativ zu der longitudinalen Achse des länglichen Elements (14) zu sein;
wobei ein Softwarealgorithmus, der auf einem Prozessor läuft, eine erste Position des länglichen Elements, eine erste Position des Endeffektors aufzeichnet und einen vorbestimmten Positionsreferenzpunkt etabliert, der eine Beziehung zwischen der ersten Position des länglichen Elements und der ersten Position des Endeffektors ist, wobei der Softwarealgorithmus weiter alle Positionsänderungen des länglichen Elements relativ zu dem Endeffektor aufzeichnet und, auf ein Bedienerkommando hin, den Endeffektor und das längliche Element repositioniert, um sie dem vorbestimmten Positionsreferenzpunkt anzugleichen.

2. Chirurgisches Instrument (10) nach Anspruch 1, wobei der Endeffektor ein Paar von Klemmbackenelementen einschließt.

3. Chirurgisches Instrument (10) nach Anspruch 1 oder Anspruch 2, wobei die Vielzahl von Richtungen vertikale Verschiebungen und horizontale Verschiebungen einschließen, wobei die Verschiebungen mittels einem oder mehreren Aktuatoren angetrieben werden, die innerhalb des Griffelements (12) positioniert sind.

4. Chirurgisches Instrument (10) nach einem vorstehenden Anspruch, wobei das Griffelement (12) eine Antriebsanordnung zum Aktuieren der gerichteten Bewegung des Endeffektors und der Rotationsbewegung des länglichen Elements (14) einschließt.

5. Chirurgisches Instrument (10) nach Anspruch 1, wobei, wenn das längliche Element (14) in eine zweite Position rotiert, der Softwarealgorithmus die zweite Position des länglichen Elements (14) aufzeichnet und die zweite Position des länglichen Elements (14) mit der ersten Position des Endeffektors vergleicht.

6. Chirurgisches Instrument (10) nach einem vorstehenden Anspruch, wobei der Softwarealgorithmus Triangulationstechniken verwendet, um den Endeffektor zu repositionieren, um einen vorbestimmten Positionsreferenzpunkt des länglichen Elements (14) anzugleichen, vorzugsweise wobei die Triangulationstechniken die Verwendung von einem oder mehreren Winkelencodern einschließen, noch bevorzugter, wobei die einen oder mehreren Winkelencoder magnetische Sensoren oder Potentiometer sind.

7. Chirurgisches Instrument (10) nach einem vorstehenden Anspruch, wobei der Softwarealgorithmus mittels eines Steuerpanels gesteuert wird, das um das Griffelement (12) positioniert ist.

8. Chirurgisches Instrument (10) nach einem vorstehenden Anspruch, wobei der Softwarealgorithmus drahtlos mittels einer externen Quelle gesteuert wird.

9. Chirurgisches Instrument (10) nach Anspruch 8, wobei die externe Quelle eine Rechnereinrichtung ist, vorzugsweise wobei die externe Quelle eine Rechnereinrichtung ist, die an einem entfernten Ort positioniert ist.

## Revendications

1. Instrument (10) chirurgical, comprenant :
un élément allongé (14) ayant une extrémité proximale et une extrémité distale, l'élément allongé (14) définissant un axe longitudinal et configuré pour tourner par rapport à l'axe longitudinal ;
un élément formant poignée (12) configuré pour être attaché à l'extrémité proximale de l'élément allongé (14), l'élément formant poignée (12) étant associé de manière opérationnelle à un mécanisme de rotation ; et
un effecteur d'extrémité configuré pour être attaché à l'extrémité distale de l'élément allongé (14), l'effecteur d'extrémité étant associé de manière opérationnelle à un mécanisme d'articulation et configuré pour pouvoir être pivoté tout au long d'une pluralité de directions par rapport à l'axe longitudinal de l'élément allongé (14) ;
dans lequel un algorithme logiciel s'exécutant sur un processeur enregistre une première position de l'élément allongé, une première position de l'effecteur d'extrémité et établit un point de référence positionnelle prédéterminé qui est une relation entre la première position de l'élément allongé et la première position de l'effecteur d'extrémité, dans lequel l'algorithme logiciel enregistre en outre tous les changements de position de l'élément allongé par rapport à l'effecteur d'extrémité et, lors d'un ordre d'opérateur, repositionne l'effecteur d'extrémité et l'élément allongé pour correspondre au point de référence positionnelle prédéterminé.

2. Instrument chirurgical (10) selon la revendication 1, dans lequel l'effecteur d'extrémité inclut un couple d'éléments formant mâchoires.

3. Instrument chirurgical (10) selon la revendication 1 ou la revendication 2, dans lequel la pluralité de directions inclut des déplacements verticaux et des déplacements horizontaux, les déplacements étant entraînés par un ou plusieurs dispositifs d'actionnement positionnés à l'intérieur de l'élément formant poignée (12).

4. Instrument chirurgical (10) selon l'une quelconque des revendications précédentes, dans lequel l'élément formant poignée (12) inclut un ensemble d'entraînement pour actionner le mouvement directionnel de l'effecteur d'extrémité et le mouvement rotatif de l'élément allongé (14).

5. Instrument chirurgical (10) selon la revendication 1, dans lequel, lorsque l'élément allongé (14) tourne jusqu'à une seconde position, l'algorithme logiciel enregistre la seconde position de l'élément allongé (14) et compare la seconde position de l'élément allongé (14) à la première position de l'effecteur d'extrémité.

6. Instrument chirurgical (10) selon l'une quelconque des revendications précédentes, dans lequel l'algorithme logiciel utilise des techniques de triangulation pour repositionner l'effecteur d'extrémité pour correspondre à un point de référence positionnelle prédéterminé de l'élément allongé (14), de préférence dans lequel les techniques de triangulation impliquent l'utilisation d'un ou plusieurs codeurs angulaires, de préférence toujours dans lequel le(s) un ou plusieurs codeurs angulaires sont des capteurs magnétiques ou des potentiomètres.

7. Instrument chirurgical (10) selon l'une quelconque des revendications précédentes, dans lequel l'algorithme logiciel est commandé via un panneau de commande positionné autour de l'élément formant poignée (12).

8. Instrument chirurgical (10) selon l'une quelconque des revendications précédentes, dans lequel l'algorithme logiciel est commandé sans fil via une source externe.

9. Instrument chirurgical (10) selon la revendication 8, dans lequel la source externe est un dispositif informatique, de préférence dans lequel la source externe est un dispositif informatique positionné dans un emplacement distant.
